(19) Europäisches Patentamt
European Patent Office
Office européen des brevets

(11) **EP 4 208 251 B1**

(12) **EUROPEAN PATENT SPECIFICATION**

(45) Date of publication and mention
of the grant of the patent:
**02.07.2025 Bulletin 2025/27**

(21) Application number: **21769457.9**

(22) Date of filing: **01.09.2021**

(51) International Patent Classification (IPC):
***A61N 1/372*** *(2006.01)*    ***A61N 1/378*** *(2006.01)*

(52) Cooperative Patent Classification (CPC):
**A61N 1/37276; A61N 1/37217; A61N 1/3727;
A61N 1/3787**

(86) International application number:
**PCT/EP2021/074099**

(87) International publication number:
**WO 2022/049107 (10.03.2022 Gazette 2022/10)**

(54) **IMPLANTABLE MICRO DEVICE WITH HIGH DATA RATE BACK SCATTERING**

IMPLANTIERBARE MIKROVORRICHTUNG MIT HOHER DATENRATENRÜCKSTREUUNG

MICRO DISPOSITIF IMPLANTABLE AYANT UNE RÉTRODIFFUSION À HAUT DÉBIT DE DONNÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **01.09.2020   EP 20193851**

(43) Date of publication of application:
**12.07.2023   Bulletin 2023/28**

(73) Proprietor: **Aarhus Universitet
8000 Aarhus C (DK)**

(72) Inventors:
• **RASHIDI, Amin
  2628 ZG Delft (NL)**
• **MORADI, Farshad
  8240 Risskov (DK)**
• **ZAMANI, Milad
  8240 Risskov (DK)**

(74) Representative: **Høiberg P/S
Adelgade 12
1304 Copenhagen K (DK)**

(56) References cited:
**WO-A1-2020/047152    WO-A1-2020/142733
WO-A2-2018/009905    US-A1- 2019 321 640**

## Description

FIELD OF THE INVENTION

The present invention relates to the field of implantable micro devices. More specifically, the invention provides a micro device for implantation into biological tissue, e.g. the brain, such as a so-called brain dust. Specifically, the invention provides a micro device with a high data rate capability based on ultrasonic backscattering, e.g. for communicating sensed data to an external device.

BACKGROUND OF THE INVENTION

Micro devices suitable for implanting into biological tissue are typically electrically powered with one or more sensors and/or one or more actuators (light source, electric stimulators etc.). Typically, such micro devices transmit sensed data to an external device. For further development of functionalities of such micro devices, e.g. the so-called dusts for implantation into brain tissue, a high data rate for communication with the micro device is required.

In spite the very compact dimensions of such micro devices, a considerable data rate is required for transmission of e.g. neural activity data to an external device, e.g. for implementation of a brain-computer interface and/or for treatment or therapy of diseases by means of applying light and/or electric stimulation provided by the dust. Especially, if a large number of micro devices are implanted, such as tenths or hundreds, a high data rate capacity is required for each device to provide an efficient wireless data communication for real-time or near real-time transfer of data without the devices occupying the same data channels continuously.

The micro device may have a radio frequency (RF) or optical transmitter for data transmission, however high absorption in the biological tissue limits the use of these in practice due to lack of power and due to safety. Ultrasonic transmission is possible, and especially, data can be transmitted by ultrasonic backscattering utilizing the on-board piezoelectric transducer present for receiving power by an ultrasonic signal from an external transmitter. However, typically the data rate possible for such ultrasonic backscattering is rather poor due to the high wavelength and low frequency of the ultrasonic data carrier.

The data capacity should be high, while still with a low power consumption, due to the limited power available in the micro device. Thus, for very compact micro devices, such as brain dusts, there is a need for an efficient way of providing a high data rate and still with a low electric power consumption.

WO 2018/009905 discloses an implantable device having a sensor configured to detect an amount of an analyte, a pH, a temperature, strain, or a pressure; and an ultrasonic transducer configured to receive current modulated based on the analyte amount, the pH, the temperature, or the pressure detected by the sensor, and emit an ultrasonic backscatter based on the received current. The implantable device can be implanted in a subject, such as an animal or a plant.

WO 2020/142733 discloses a system for controlling power provided to a device implantable in a subject are described. A method is performed at the implantable device to receive, from an interrogator, powering ultrasonic waves having a wave power. Then, energy from the powering ultrasonic waves is converted into an electrical signal to power the implantable device.

US 2019/321640 discloses an implantable medical device including an ultrasonic transducer configured to receive ultrasonic waves and convert energy from the ultrasonic waves into an electrical energy that powers the device, two or more electrodes in electrical communication with the ultrasonic transducer that are configured to electrically stimulate a splenic nerve or detect a splenic nerve activity, and optionally a splenic nerve attachment member

WO 2020/047152 discloses implantable closed-loop neuromodulation devices, systems that includes such devices and an interrogator configured to emit ultrasonic waves that power the device, methods of using such devices and systems, and methods of modulating neural activity.

SUMMARY OF THE INVENTION

The invention is defined in the appended claims. Aspects, embodiments and examples disclosed herein which do not fall within the scope of the appended claims do not form part of the invention, and are merely provided for illustrative purposes.

Following the above, it may be seen as an object of the present invention to provide a micro device capable of transmitting sensed data to an external receiver at a high data rate, and preferably still with a minimal power consumption.

In a first aspect, the invention provides a micro device, such as a brain dust, arranged for implantation into biological tissue, the micro device comprising

- a piezoelectric transducer,
- a power management circuit connected to the piezoelectric transducer, and being arranged to generate an electric power output for powering components of the micro device in response to an ultrasonic power signal received by the piezoelectric transducer from an external source, such as the electric power output being arranged for powering power consuming components of the micro device,
- a sensor arranged to measure a physical parameter or a neural activity, such as a bio-potential signal, a biochemical signal, or a biomechanical signals, and to generate an electric signal accordingly,
- an electric circuit arranged to receive the electric signal from the sensor, and to digitize the electric signal by means of time-encoding analog-to-digital converter, such as a pulse width modulator, e.g. a delta-sigma modulator, to generate a one-bit data stream representing the electric signal from the sensor, and

- a load modulation circuit comprising at least one electric switch connected to terminals of the piezoelectric transducer, so as to allow modulation of electric load of the piezoelectric transducer in response to the one-bit data stream, so that a backscattered signal from the piezoelectric transducer is modulated by the one-bit data stream,

characterized in that
the micro device (MD) is arranged to store a time sequence of the generated one-bit data stream in a memory, and applying the stored time sequence of the one-bit data stream to the load modulation circuit at an increased data rate to provide a time compression of data represented in the backscattered signal.

It has been found that if the measured data stream is applied to the load modulation circuit with a proper bit-rate, it is possible to provide an analog modulation of the ultrasonic backscattered signal from the piezoelectric transducer in response to an ultrasonic power signal received from an external transmitter.

Such micro device is advantageous since the transmission data rate can be increased considerably by analog modulation of the backscattered signal. Furthermore, in comparison with other analog modulation of the backscattered signal, modulation depth can be increased by switching the backscattered signal between a near minimum (when electrical load is set to achieve a good matching and least reflection) to a near maximum (when changing the load cause a very poor matching and consequently maximum reflection). It is helpful to get higher signal to noise ratio at the ultrasonic receiver.

It has been found that the bandwidth of ultrasonic backscattering link can be calculated by measuring the transient response of the backscattered signal to a step function at the load of the micro-device. Thus, the whole bandwidth of link can be efficiently used by compressing the measured data at time domain which results in expansion of the signal in frequency domain. For example, a low bandwidth neural activity signal e.g. local field potential can be transmitted in a time-compressed version, thus allowing transmission of real-time data with intermediate periods available for transmission of other data from the micro device, and/or for transmission of data on the same ultrasonic carrier frequency by other micro devices.

Further, using a delta-sigma modulator and a simple switching circuit allows the backscatter modulation to be implemented by rather simple components that occupy minimal extra volume and only consume a small amount of extra power.

In the following, preferred features and embodiments of the first aspect will be described.

The load modulation circuit is preferably arranged to control the at least one electric switch according to the one-bit data stream at a modulation frequency. The choice of modulation frequency depends on a number of parameter, and a design methodology will be described later.

Most preferably, the micro device is arranged to store a time sequence of the generated one-bit data stream in a memory, and applying the stored time sequence of the one-bit data stream to the load modulation circuit at an increased data rate to provide a time compression of data represented in the backscattered signal. Especially, the increased data rate can be at least a factor of 5, such as at least a factor of 10, e.g. 10-50, compared to a data rate of the one-bit data stream generated by the time-encoding analog-to-digital converter. In this way, e.g. a low-bandwidth sensor signal can be transmitted in near real time occupying only a fraction of the available data transmission time. Especially, the stored time sequence may be chose to have a length of 1-1000 ms, such as 1-500 ms, such as 5-200 ms, such as 5-100 ms. In general, the choice may depend on a number of factors, e.g. the bandwidth of the sensor signal to transmit. A design methodology will be further elaborated on later.

The micro device is preferably arranged to store measured data from the sensor continuously over a certain period of time, and to recall the stored data and apply the data to the load modulation circuit during a period of communication.

In some embodiments, the load modulation circuit comprises at least two electric switches connected to the piezoelectric transducer and arranged for being controlled to modulate electric load of the piezoelectric transducer in response to the one-bit data stream.

Preferably, the load modulation circuit is arranged to control electric load of the piezoelectric transducer between a first load state and a second load state in response to the one-bit data stream, wherein the at least one electric switch is controlled so as to provide different electric loads of the piezoelectric transducer in the first load state than in the second load state. Especially, in the first load state the at least one electric switch is controlled to short-circuit the terminals of the piezoelectric transducer. Especially, in the second load state the at least one electric switch is controlled to provide an electric load of the terminals of the piezoelectric transducer to cause a minimal backscattering from the piezoelectric transducer. In this way, the optimal ultrasonic signal amplitude can be obtained, since the piezoelectric transducer is switched between load states causing minimal and maximal backscattering. This allows a high signal to noise ratio experienced at the receiver side (interrogator).

The time-encoding analog-to-digital converter is preferably arranged to sample the electric signal from the sensor at an oversampling rate of at least a factor of 64 of a bandwidth of the electric signal, such as at least a factor of 128, such as at least a factor of 256.

The sensor is one of: a neural activity sensor such as a Local Field Potential sensor or a single cell sensor, a temperature sensor, a pressure sensor or a bio-chemical sensor. Especially, the micro device may comprise a plurality of sensors, e.g. a plurality of the mentioned types of sensors. Preferably, with such plurality of sensors, the load modulation circuit may be arranged to alternately modulate electric load of the piezoelectric transducer in response to one-bit data stream sequences from the plurality of sensors. In this way, a backscattered signal from the piezoelectric transducer will be modulated by one-bit data from the plurality of sensors distributed in time. Especially, one-bit data sequences from the plurality of sensors can be stored and applied to the load modulation circuit at a higher data rate than originally obtained, thus providing a time-compressed data transmission of near real time data from the plurality of sensors.

The sensor, e.g. a neural activity sensor, may generate an electric signal with a bandwidth of below 500 Hz, e.g. below 200 Hz, such as within 10-200 Hz, or such as within 50-150 Hz e.g. as it is the case for a neural activity sensor. It is to be understood that other sensors may generate an electric signal with a much lower bandwidth, e.g. a temperature sensor or the like, and such sensors therefore require significantly smaller data rate for transmission. Time compression of such data allows to send them in a higher data rate to exploit the full bandwidth of the link in an efficient way.

In some embodiments, the micro device comprises a processor capable of executing a processing algorithm to receive data from the sensor and to generate an event based one-bit data stream accordingly, (e.g. the spikes generated by the neuromorphic computing circuits) such as a neural activity event based one-bit data stream, and wherein the event based one-bit data stream is applied to the modulator circuit for transmission as a backscattered signal.

In a specific embodiment, the micro device is configured for implantation into brain tissue, thus being a so-called brain dust.

In preferred embodiments, the power management unit and the load modulation circuit are implemented on an integrated circuit die, such as the micro device, or dust, having outer dimensions occupying a total volume of less than 5 mm$^3$, such as less than 2 mm$^3$ or even less than 1 mm$^3$.

In some embodiments, the micro device comprises one or more controllable actuators for providing stimulus to surrounding biological tissue. Such controllable actuators may comprise one or more controllable light sources for providing light to surrounding biological tissue, and/or one or more controllable electric stimulators with electrodes in contact with surrounding biological tissue. For controlling the one or more controllable actuators, the micro device may comprise a wireless receiver arranged to receive data from an external device for controlling the at least one controllable actuator, e.g. to control switch on/off of the individual actuator and/or to control intensity of the actuator (light intensity or electrical stimulation intensity and/or frequency etc. The wireless receiver may be an RF receiver or an ultrasonic receiver, especially the piezoelectric transducer may be arranged to receive an ultrasonic control signal for controlling function of one or more actuators in the micro device.

Especially, the at least one controllable light source may be arranged for optogenetic stimulation and/or photodynamic therapy, and/or optic triggering of release of a drug to the biological from a drug container which may be arranged inside or outside the micro device. Specifically, the micro device may comprise first and second controllable light sources arranged to generate light at different wavelengths, such as the first and second controllable light sources being arranged for controllable optogenetics or photodynamic therapy at different wavelengths of light. Especially, the first controllable light source is arranged for one of: controllable optogenetics, photodynamic therapy or for optical triggering of drug delivery, and wherein the second controllable light source is arranged for one of: controllable optogenetics, photodynamic therapy or for optical triggering of drug delivery. Even more specifically, the micro device may comprise a third controllable light source arranged for controllable optogenetics, photodynamic therapy or for optical triggering of drug delivery. Especially, the first and second controllable light sources are then arranged for controllable optogenetics or photodynamic therapy at different wavelengths of light, and wherein the third controllable light source is arranged for optical triggering of drug delivery.

As a further option, the micro device may comprise at least one controllable light source and an electrode arranged for controllable electric stimulation of biological tissue, and wherein the at least one controllable light source is arranged for controllable optogenetics or photodynamic therapy, such as for providing hybrid light and electric stimulation of biological tissue.

It is to be understood that the choice of piezoelectric transducer for the micro device as well as choice of ultrasonic frequency for the ultrasonic power signal etc. is known by the skilled person within micro electronics.

In an advantageous embodiment of the invention, the size of the micro device is small, and for implantation purposes, it may be preferred that the micro device is as small as possible. In preferred embodiment, the dimensions of the micro device is within 1x1x1 mm (height x length x width), such as within 500x500x500 μm, such as within 400x400x400 μm, such as within 300x300x300 μm, such as within 200x200x200 μm and in some embodiments it may be seen as most preferably to be within 100x100x100 μm. It is to be understood that the micro device may preferably be even smaller than 100x100x100 μm in case the actual manufacturing technologies chosen allows to.

In preferred embodiments, the micro device has a total volume of less than 2 mm³, preferably less than 1 mm³, preferably less than 0.7 mm³, such as less than 0.5 mm³.

In some embodiments, the micro device have non-uniform height, length and width. Especially, the height, length, and width dimensions may be such as 200x150x100 μm, or such as 150x150x100 μm, or such as the micro device having a height within 0.5-1.5 mm, a length of 0.5-1.0 mm, and a width of 0.3-0.7 mm.

In a second aspect, the invention provides a neural sensor system comprising

- a micro device according to the first aspect,
- an ultrasonic transmitter arranged to transmit an ultrasonic power signal to the micro device, and
- an ultrasonic receiver (or interrogator) arranged to receive the backscattered signal from the piezoelectric transducer of the micro device, and to de-modulate the backscattered signal, such as involving low-pass filtering the backscattered signal, to arrive at a representation of a time sequence of the physical parameter measured by the sensor in the micro device.

Especially, the sensor may comprise a neural activity sensor, e.g. a Local Field Potential (LFP) sensor, or the like, to sense neural activity. Such micro device may, if configured for implementation into brain tissue, to form part of a computer-to-brain interface. Especially, the micro device may comprise a controllable electrical stimulator for electrical stimulation and/or a controllable light source for optical stimulation of the brain tissue. In such embodiment, the system may form part of a closed loop computer-brain interface.

Especially, the sensor system may comprises a plurality of micro devices according to the first aspect, wherein the sensor in each of the plurality of micro devices comprises a neural activity sensor, and wherein the ultrasonic receiver (or interrogator) is arranged to receive backscattered signals from the plurality of micro devices, and to de-modulate the backscattered signals to arrive at representations of respective time sequences of neural activities measured by the plurality of micro devices. Especially, the plurality of micro devices, or dusts, may be distributed at various locations or parts of the brain. Such sensor system can be used to monitor brain activity and thus form part of a computer-brain interface. If further the plurality of micro devices, or dusts, comprises a controllable electric and/or optical stimulator for stimulation of the brain tissue, the sensor system may form part of a closed loop computer-brain interface.

In a third aspect, the invention provides a method for transmitting sensor data from a micro device implanted in biological tissue, the method comprises

- providing a micro device comprising a piezoelectric transducer connected to a power management circuit for powering power consuming components of the micro device by means of an ultrasonic power signal received by the piezoelectric transducer from an external source, the micro device further comprising a sensor arranged to measure a physical parameter and to generate an electric signal accordingly,
- digitizing the electric signal from the sensor to generate a one-bit data stream being a representation of the electric signal from the sensor, and
- modulating electric load of the piezoelectric transducer in response to the one-bit data stream, so that a backscattered signal from the piezoelectric transducer is modulated by the one-bit data stream,
- storing a time sequence of the generated one-bit data stream in a memory, and applying the stored time sequence of the one-bit data stream to a load modulation circuit at an increased data rate to provide a time compression of data represented in the backscattered signal.

Especially, the micro device according to the first aspect or the system according to the second aspect may be used for measuring neural activity in a living person or animal to provide data to diagnose one or more abnormalities or diseases, or to provide data to assist a medical doctor in such diagnose.

The same advantageous mentioned for the first aspect apply for the second and third aspects as well. The individual aspects of the present invention may each be combined with any of the other aspects. These and other aspects of the invention will be apparent from the following description with reference to the described embodiments.

BRIEF DESCRIPTION OF THE FIGURES

The invention will now be described in more detail with regard to the accompanying figures. The figures show one way of implementing the present invention and is not to be construed as being limiting to other possible embodiments falling within the scope of the attached claim set.

FIG. 1a illustrates a block diagram of a micro device embodiment, while FIG. 1b illustrates a block diagram of a sensor system embodiment,
FIG. 2 illustrates steps of a method embodiment,
FIG. 3 illustrates an example of a load modulation circuit,
FIG. 4 illustrates graphs showing a simulation of the proposed analog backscattering,
FIG. 5 illustrates a graph showing acoustic intensity at the piezoelectric transducer in response to a step input of load modulation at the micro device,
FIG. 6 illustrates a diagram of an example of a SRAM memory with peripherals and interfaces,
FIG. 7 illustrates an example of a control circuit for the SRAM memory of FIG. 6, and

FIG. 8 illustrates steps of an example of a design methodology for optimal utilization of an ultrasonic backscattering data channel.

## DETAILED DESCRIPTION OF AN EMBODIMENT

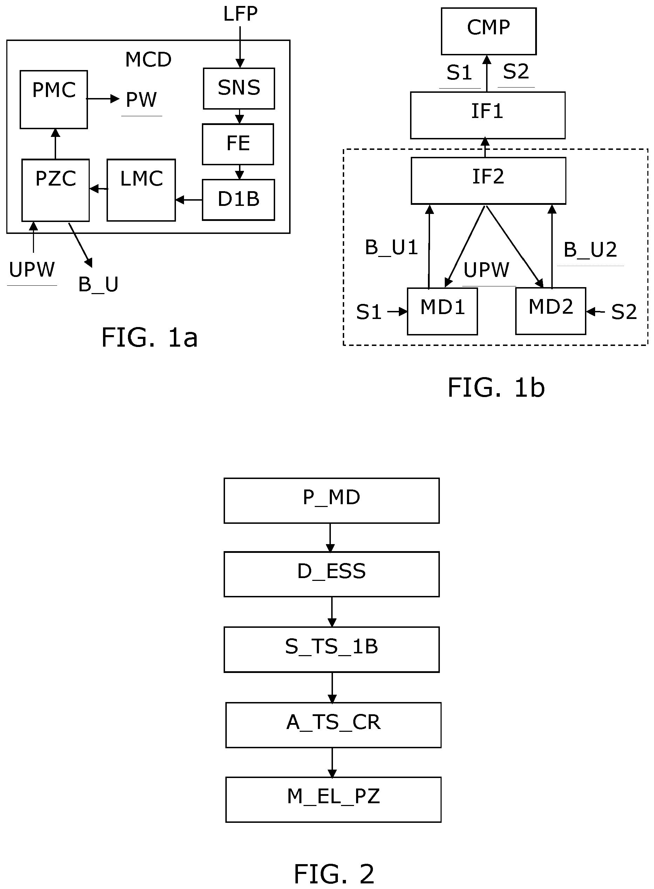

FIG. 1a illustrates a micro device MD, e.g. a dust, embodiment which receives an ultrasonic power signal UPW from an external source by means of a piezoelectric crystal PZC. A power management circuit PMC is connected to electric terminals of the piezoelectric crystal PZC and generates a power output PW accordingly for powering the power consuming components of the micro device MD. A sensor SNS serves to measure a physical parameter related to the biological tissue in which the micro device MD is implanted, e.g. a neural activity signal, such as a Local Field Potential LFP. The sensor SNS generates an electrical signal according to the measured parameter, and this is received by a front end circuit FE which may comprise a pre-amplifier with a high impedance, and optionally further conditioning circuits. The amplified signal from the sensor is then applied to a time-encoding analog-to-digital converter D1B, e.g. a delta-sigma modulator, which generate a one-bit data stream representing the electric signal from the sensor.

The one-bit data stream is applied to a load modulation circuit LMC with one or more electric switches connected to terminals of the piezoelectric transducer PZC, so as to allow modulation of electric load of the piezoelectric transducer PZC in response to the one-bit data stream, preferably a harsh switch of load to provide a significant load change following the one-bit data stream. Hereby, the piezoelectric transducer PZC will generate a backscattered signal B_U in response to the received ultrasonic power signal UPW which is modulated by the one-bit data stream. Thus, the backscattered ultrasonic signal B_U from the micro device MD contains data generated by the sensor SNS, and an external ultrasonic receiver can pick up this signal B_U and perform a de-modulation to arrive at data representing a time signal sensed by the sensor SNS in the micro device MD. In this way it has been found that a high data rate can be obtained in the transmission of data from the micro device MD. Further, this way of transmitting data only requires a minimum of extra components in the micro device MD, e.g. a delta-sigma modulator, while the piezoelectric crystal PZC component is used for power harvesting as well as data transmission. Thus, with the proposed method for data transmission a high data rate is combined with a low power consumption and a low volume required for extra components.

It is so be understood that the micro device MD may comprise further components with other functionalities, e.g. one or more light sources (micro LEDs) and further sensors. A wireless receiver may be used to receive wireless control signals for controlling such other components. E.g. for controlling light sources for generating light to the biological tissue for optogenetics and/or, and/or for optically triggering of drug delivery to surrounding biological tissue by providing light on a drug container inside or outside the micro device MD.

FIG. 1b illustrates a block diagram of a sensor system embodiment for use as a computer-brain interface The dashed box indicates components implanted inside the skull of a person. In this embodiment, a three layer approach is provided for communication and powering of two implantable brain dusts MD1, MD2 which each has a piezoelectric transducer and a sensor. The sensors sense respective neural activity time signals S1, S2. Both ultrasonic power signal UPW and generate respective backscattered ultrasonic signals B_U1, B_U2 in which representations of the sensed neural activity time signals S1, S2 are contained according to the described analog modulation principle.

A computer CMP outside a person's body is an end-receiver of the neural activity time signal data S1, S2 sensed by the micro device MD1, MD2. A first interface part IF1 is arranged for position outside the skull, on the head, of a person, and this first interface part IF1 serves as interface between the computer CMP and a second interface part IF2. The second interface part IF2 is arranged for implantation inside the skull of the person and serves as interface between the first interface part IF1 and the micro devices MD1, MD2. The second interface part IF2 has an ultrasonic transmitter which transmits the power signal UPW for powering the micro devices MD through the brain tissue. Further, the second interface part IF2 comprise an interrogator part arranged to receive the backscattered ultrasonic signals B_U1, B_U2 through the brain tissue from the micro device MD1, MD2. The first and second interface parts IF1, IF2 are connected by a wireless transmission, e.g. an ultrasonic and/or a radio frequency (RF). A de-modulation processing, including a low-pass filtering, to de-modulate the received backscattered signals B_U1, B_U2, may be performed in the computer CMP, the first interface part IF1 or the second interface part IF2, preferably in the first or second interface parts.

The first and second interface parts IF, IF2 may further be arranged to communicate wireless control signals for controlling function of the micro devices MD1, MD2, e.g. via ultrasonic signal or via electromagnetic RF signals. Such functions of the micro devices MD1, MD2 may be stimulation of the brain tissue by means of optical and/or electrical signals and/or drug to be controllably released by the micro device. In this way, a computer-brain interface can be implemented, e.g. as a closed-loop control system controlled by a control algorithm in the CMP.

FIG. 2 illustrates steps of an embodiment of a method for transmitting sensor data from a micro device, e.g. a dust, implanted in biological tissue, e.g. brain tissue. First, providing P_MD a micro device (a dust) comprising a piezoelectric transducer connected to a power management circuit for powering power consuming components of the micro device by means of an ultrasonic power signal received by the piezoelectric transducer from an external source, the micro device further comprising a sensor arranged to measure a physical parameter and to generate an electric signal accordingly. Next, digitizing D_ESS the electric signal from the sensor to generate a one-bit data stream being a representation of the electric signal from the sensor. Next, storing S_TS_1B a time sequence of the one-bit data stream in a memory in the micro device, e.g. a time sequence having a length of 1-100 ms in case of a neural activity sensor. Next, applying A_TS_CR the stored time sequence of one-bit data stream to a load modulation circuit which finally performs modulating M_EL_PZ electric load of the piezoelectric transducer in response to the applied one-bit data stream. Hereby, a resulting backscattered signal from the piezoelectric transducer is modulated by the one-bit data stream. Thus, it is to be understood that the method preferably comprises the step of receiving an ultrasonic power signal by the piezoelectric transducer from an external ultrasonic power source, thus receiving an ultrasonic signal which is then backscattered in a modulated version to represent data indicative of the signal generated by the sensor in the micro device.

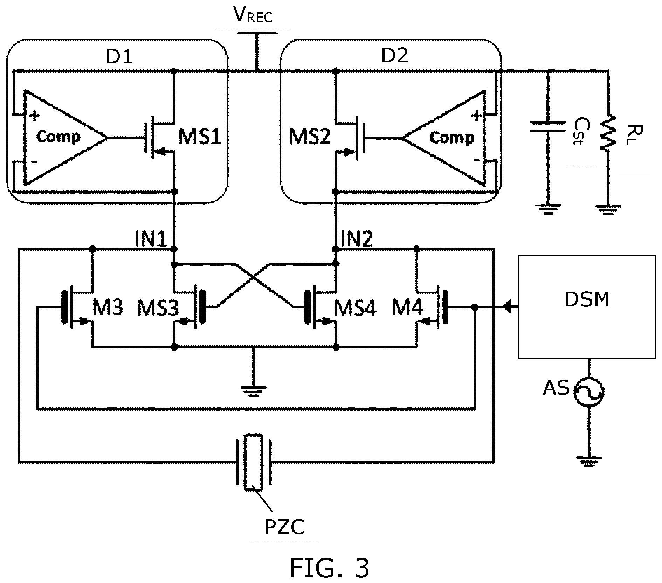

FIG. 3 illustrates an example of a load modulation circuit. An analog signal AS from a sensor is applied to a delta-sigma modulator DSM which generates a 1-bit data stream output controlling a switch arrangement with electric switches M3 and M4 connected to electrical terminals of the piezoelectric crystal PZC for switching its electric load in response to the 1-bit data stream. M3 and M4 are the load modulation circuit in FIG. 3.

In essence, this circuit modulates the piezoelectric crystal's PZC electrical load harshly according to each bit of the digitized 1-bit data stream by connecting it to either:

1) an optimum load, i.e. the load that results a minimum ultrasonic reflection from the dust, or
2) zero-impedance load, i.e. shortening of the crystal's terminal that results in maximum ultrasonic reflection.

In this circuit, the transistor M3 and M4 in conjunction with active diodes D1 and D2 are building blocks of an active rectifier that converts the AC signal at the terminals of the PZC to a DC voltage for the power consuming elements modeled by a resistive load $R_L$. $C_{St}$ is the storage capacitor at the output of the rectifier. The rectifier followed by its loads should results in an optimum load leading to a minimum reflection from the PZC.

With the proposed modulation, the push of backscattered signal has a low-pass response to the proposed modulations and filters out most of the quantization noise added by the delta-sigma modulator DSM and results in appearance of the analog signal over the push of the backscattered waves. Since, the load and consequently the backscattered signals are modulated harshly in this approach, the amplitude of the modulates signal will be maximized that can results in higher signal to noise ratio of the signal at the interrogator.

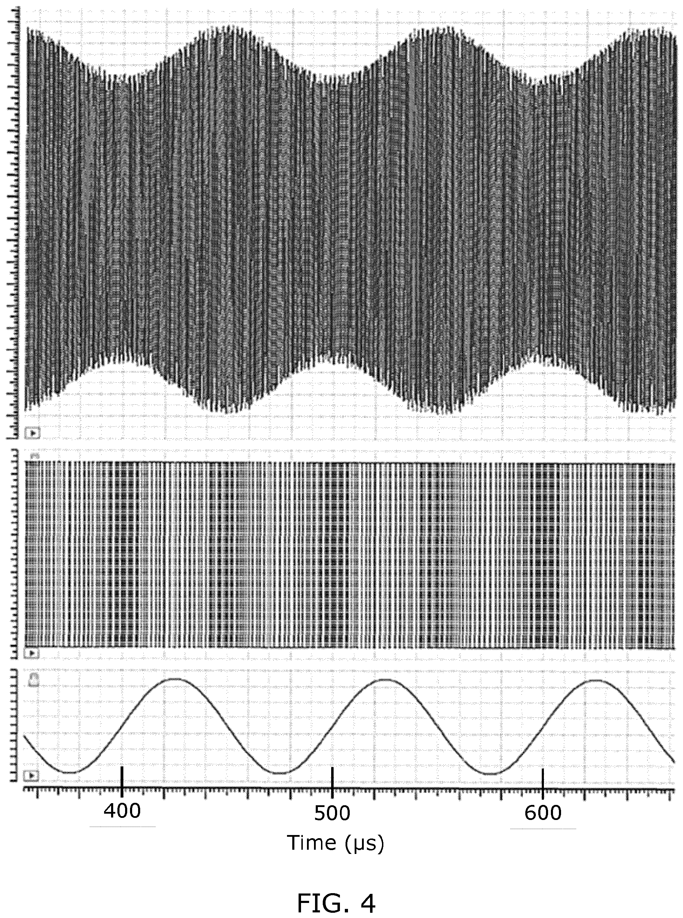

FIG. 4 illustrates graphs showing a simulation of the proposed analog backscattering, namely three graphs indicating amplitude versus time. A verilog-A model for modeling a first-order delta-sigma modulator is used.

The bottom graph shown an analog 10 kbit sinusoidal input signal, as an example of a sensor analog electric signal.

A 2 MHz clock to the delta-sigma modulator has been used for sampling, and the middle graph shows the digital output of the delta-sigma modulator.

The output of the delta-sigma modulator block was fed into a the load modulation circuit (Fig. 3), and the simulated result for the modulated backscattered signal is shown in the upper graph. As seen, the analog backscattering modulation is easily seen, and it has been found that it is possible to de-modulate at the interrogator side, including a low-pass filtering, to arrive at a representation of the original analog input signal with an acceptable signal-to-noise ratio.

In order to be able to utilize an increase data rate which is possible with the proposed analog modulation backscattering approach for low-bandwidth signals, it can preferably be combined with time compression of the data representing time signal from the sensor, and still allow transmission of the time signal data in real time or at least near real time. This can be obtained by time compression. This can be obtained by storing a time sequence of the sensor output in a 1-bit represenation, e.g. an LFP signal having a bandwidth of about 100 Hz, which has been sampled with a reasonable oversampling rate, e.g. 128. By applying recalling the stored 1-bit data stream and applying it to the load modulation circuit at an increased data rate, e.g. with much higher frequency e.g. 100-200 times, a time compression is obtained, and thus e.g. 10 ms of time signal can be transmitted in the backscattered representation in less than 100 μs. Thus, the data capacity can be utilized to transmit time data from additional sensors in a micro device, or it can be used as silent periods to allow other micro device to transmit data over the same ultrasonic channel without interference.

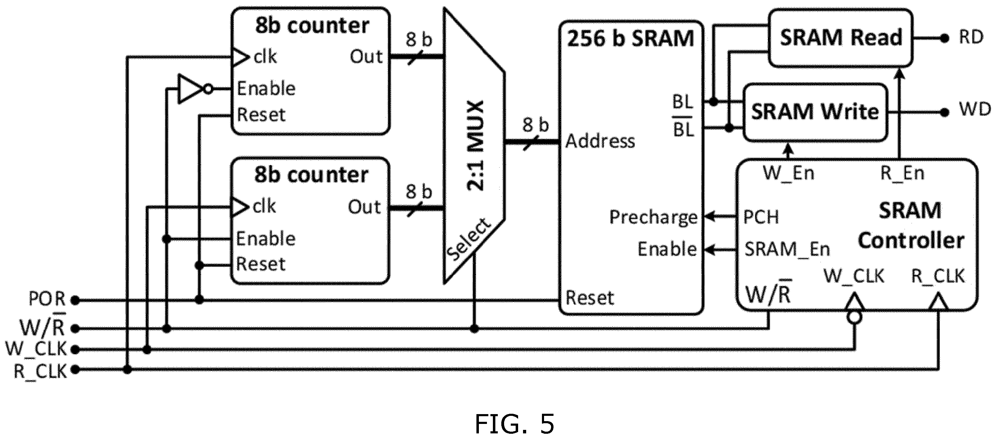

FIG. 5 illustrates a diagram of an example of a SRAM memory with peripherals and interfaces for implementing such time compression. In the diagram W-CLK and R_CLK are the clock signals that generate timings for writing and reading to the SRAM, respectively. There is one dedicated counter for addressing the SRAM cell for each of the writing and reading operations. Both of the counters get reseat using Power-On-Reset (POR) signal when the micro device, or dust, gets enough power to start working. An 8-bit two-to-one multiplexer is responsible for connecting one of the aforementioned addresses to the SRAM. The SRAM Write and SRAM Read blocks are designed to write or read from the SRAM cells, respectively. Finally, an SRAM Controller generates the controlling signals for writing/reading to/from SRAM.

One problem that needs to be addressed here is the fact that writing and reading events are not synchronized and they may happen at the same time. Writing in SRAM should be done after each sampling by the sigma-delta modulator. So, for LFP signal with a bandwidth of such as 100 Hz and with an oversampling rate of 128, writing to the SRAM is performed with a frequency of 12.8 kHz and a period of 78.125 $\mu$s. On the other hand, reading from the SRAM occurs with the same frequency as the ultrasonic power carrier, e.g. around 3 MHz. If it is intended to receive the backscattered signal in a silent time slot, the duration of a power burst should be equal to two Time of Flight (ToF) of ultrasonic waves in the brain tissue from the UPIB to the dust, which for example is about 40 $\mu$s for a distance of 3 cm. Thus, during the 40 $\mu$s of reading and load modulation, a maximum of one writing event may happen (writing events are every 78.125 $\mu$s). Furthermore, reading has a higher priority in comparison with a writing operation.

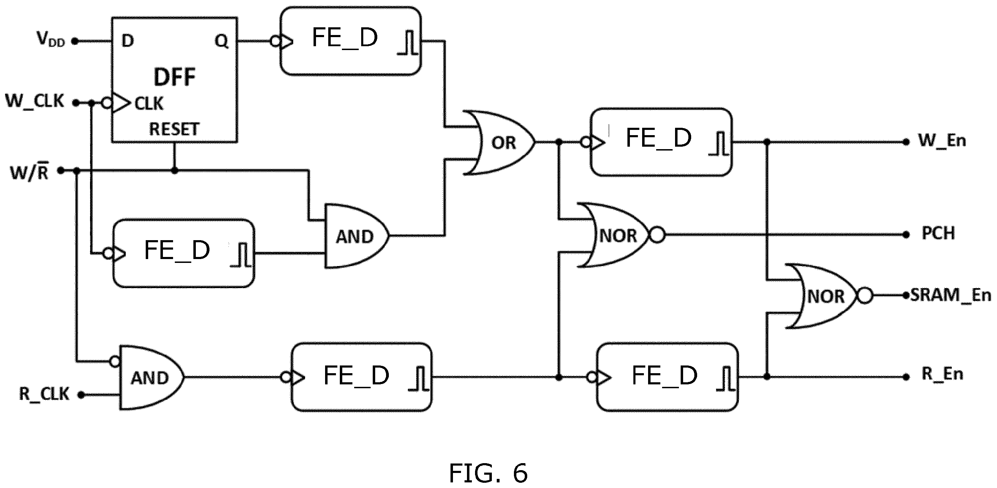

FIG. 6 shows a control circuit which solves this, namely a control circuit designed to generate output signals of Write Enable (Wr_En), Read Enable (R_En), PCH (Pre-Charge), and Decoders' Enable (Dec_En), according to input signals Write Clock(W_CLK), Read Clock (R_CLK) and Write/Read signal (W/R̄). The control circuit is formed by logic elements, a D-type flip-flop DFF, and falling edge detectors FA_D.

The DFF is reset during the writing period (when we do not read from the SRAM). Thus, at the beginning of the reading period, Q at the output of the DFF has a low logic value. Then if during the reading period, a writing event happens (falling edge of W_CLK) then the Q switches to the high logic value and again is reset at the end of the reading period. In this case, the falling edge at the output of the DFF will be detected using a falling edge detector and initiates a writing operation. In the following a design methodology is discussed for linking choice of various parameters in the design of a system implementing the proposed analog modulation ultrasonic backscattering for data transmission.

By applying a step to the load modulation circuits, the bandwidth of the backscattering link can be measured using the transient response and the rising time of the backscattered signal.

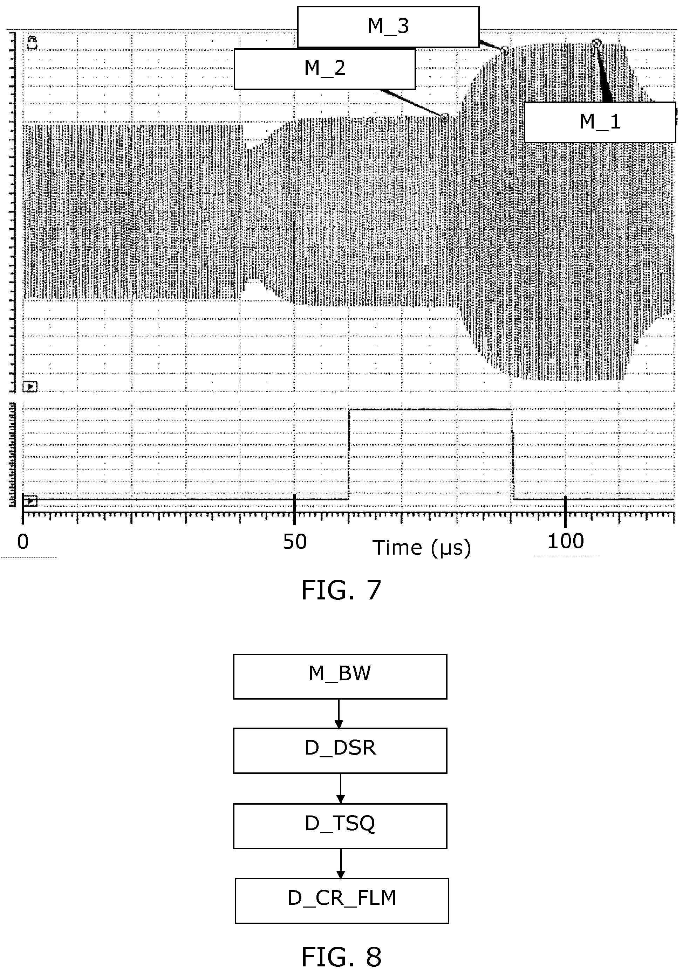

FIG. 7 illustrates graphs showing an example of a step load modulation at the dust and the backscattered signal at the external interrogator, namely in the upper graph acoustic intensity at the piezoelectric transducer versus time in response to a step input of load modulation, shown in the lower graph. The results are simulated results. The measurement points illustrates are M_1: 187 mV@106 $\mu$s, M_2: 105 mV@78 $\mu$s, and M_3: 180 mV@89 $\mu$s.

As seen, the push of the acoustic intensity at the transducer in response to a step input of load modulation at the dust is similar to a first-order low-pass filter, and thus its time function can be driven as below:

$$I(t) = I0(1 - e^{-t/\tau})$$

Where I(t) is the acoustic intensity at the transducer and I0 is its steady state value without any load modulation at the dust. Thus, the time constant of the link ($\tau$), can be calculated by measuring the rising time of the backscattered push. Rising time (tr) is when I(tr) reaches 90% of its initial value and tr=2.23 $\tau$. Thus, based on the simulation in FIG. 7, the time constant and the 3 dB link bandwidth LBW can be derived as below:

$$LBW = \frac{1}{2\pi\tau}$$

In an experiment, the ultrasonic link can be characterized in a similar way. If the bandwidth of the target signal for measurement at the dust is denoted SBW. Then, for time encoding of the data, i.e. converting the data to a 1-bit data stream, sampling with a higher rate than Nyquist rate is required. Over-sampling rate (OSR) of the data has a direct influence in the Signal to Noise Ratio (SNR) of the digitized data. In this way, the sampling rate of the data is given by DSR=2$\times$OSR$\times$SBW.

It is proposed to store the 1-bit data stream with the rate of DSR. Storing a measured data with time-duration of Tstr, requires a memory with a capacity of at least DSR$\times$ Tstr. By using the noise shaping technique, the quantization noise will be moved to the higher frequencies close to DSR/2. However, still the data is in the frequency band of SBW. For taking advantage of the whole link bandwidth efficiently, it is proposed to compress the signal in time domain which is equivalent to expanding the signal in frequency domain. Therefore, the maximum Compression Ratio (CR) to expand the data, excluding the quantization noise, over the whole link bandwidth is CR=LBW/SBW.

In this way, the noise bandwidth will be increased with the same ratio but out of LBW. Thus, the noise can be attenuated by the link. Attenuation of the quantization noise over the backscattered signal will lead to appearing the analog waveform of the signal over the push of the backscattered signal. The quantization noise can be further removed at the receiver for improving the SNR.

Furthermore, the proposed time compression is beneficial since the whole stored data can be transmitted in a shorter time and consequently by consuming less energy. Therefore, if the duration of the recorded data is Tstr, the compressed data duration will be Tcmp= Tstr/CR. For doing the time compression with the ratio CR, the data is read from the memory and applied to the load modulation circuit, with a clock frequency of fLM=DSR$\times$CR. By taking the proposed approach, the link capacity (C) can be calculated using the Shannon equation:

$$C = B \times Log_2(1 + SNR)$$

Here, B is the bandwidth of the transmitted signal over the link. As suggested by this relationship, by expanding the bandwidth of the data to the LBW using the proposed time compression, the capacity of the link can be maximized. The SNR of the signal can be improved with a proper OSR and usage of noise shaping. However, increasing the OSR requires a bigger memory, and more advanced noise shaping can increase the complexity of the circuit implementation. Thus, increasing of the link capacity by improving the SNR of the signal comes at the price of higher power consumption and silicon area.

FIG. 8 illustrates four steps of a possible design methodology for exploiting the full-bandwidth of an ultrasonic back-scattering channel according to the invention:

> 1) Measuring M_BW the bandwidth of the backscattered Link (i.e. LBW) by applying a step function to the dust's load and measuring the rising time of the backscattered signal at the external transducer.
> 2) Defining D_DSR the data sampling rate (DSR) by designing of the oversampling rate (OSR) and noise shaping circuits according to the SNR requirements. DSR=2×OSR×SBW, where SBW is the signal bandwidth.
> 3) Defining D_TSQ the time duration of the signal that is measured and stored before each transmission cycle (Tstr), and allocating enough memory (i.e. DSR× Tstr) for storing the data.
> 4) Determining D_CR_FLM he compression ratio and load modulation frequency will be driven by CR=LBW/SBW and FLM=DSR×CR, respectively.

To sum up, the invention provides an implantable micro device, or dust, with a piezoelectric transducer connected to a power management circuit which provides an electric power output for powering components of the micro device based on an ultrasonic power signal from an external ultrasonic signal source. Hereby the micro device is powered. A sensor measures a physical parameter, e.g. a neural activity signal, and generates an electric signal, which is digitized by a time-encoding analog-to-digital converter, e.g. a delta-sigma modulator, to generate a one-bit data stream representing the sensed physical parameter. A load modulation circuit with one or more electric switches connected to the piezoelectric transducer serves to modulate electric load of the piezoelectric transducer according to the one-bit data stream, thus causing a backscattered signal from the piezoelectric transducer to be modulated by the sensed physical parameter. Preferably, the piezoelectric transducer's electric load is harshly modulated by connecting it to either an optimum load for minimum reflection, or short-circuiting for maximum reflection according to each bit of the digitized data stream. Such analog modulation of the data collected at the dust over the backscattered signal to an ultrasonic interrogator increases the ultrasonic data rate capacity. This allows e.g. transmission of time-compressed data from the sensor via ultrasonic backscattering.

Although the present invention has been described in connection with the specified embodiments, it should not be construed as being in any way limited to the presented examples. The scope of the present invention is to be interpreted in the light of the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

**Claims**

1. A micro device (MD), arranged for implantation into biological tissue, the micro device (MD) comprising

> - a piezoelectric transducer (PZC),
> - a power management circuit (PMC) connected to the piezoelectric transducer, and being arranged to generate an electric power output (PW) for powering components of the micro device (MD) in response to an ultrasonic power signal (UPW) received by the piezoelectric transducer (PZC) from an external source,
> - a sensor (SNS) arranged to measure a physical parameter or a neural activity (LFP), and to generate an electric signal accordingly,
> - an electric circuit (FE) arranged to receive the electric signal from the sensor, and to digitize the electric signal by means of time-encoding analog-to-digital converter (D1B), to generate a one-bit data stream representing the electric signal from the sensor, and
> - a load modulation circuit (LMC) comprising at least one electric switch (M3, M4) connected to terminals of the piezoelectric transducer (PZC), so as to allow modulation of electric load of the piezoelectric transducer (PZC) in response to the one-bit data stream, so that a backscattered signal (B_U) from the piezoelectric transducer (PZC) is modulated by the one-bit data stream,

**characterized in that**

the micro device (MD) is arranged to store a time sequence of the generated one-bit data stream in a memory, and applying the stored time sequence of the one-bit data stream to the load modulation circuit at an increased data rate to provide a time compression of data represented in the backscattered signal.

2. The micro device according to claim 1, wherein the load modulation circuit is arranged to control the at least one electric switch according to the one-bit data stream at a modulation frequency.

3. The micro device according to claim 1, wherein the increased data rate is at least a factor of 5, compared to a data rate of the one-bit data stream generated by the time-encoding analog-to-digital converter.

4. The micro device according to claim 1, wherein the micro device is arranged to store measured data from the sensor continuously over a certain period of time, and to recall the stored data and apply the data to the load modulation circuit during a period of communication.

5. The micro device according to any of the preceding claims, wherein the load modulation circuit comprises at least two electric switches connected to the piezoelectric transducer and arranged for being controlled to modulate electric load of the piezoelectric transducer in response to the one-bit data stream.

6. The micro device according to any of the preceding claims, wherein the load modulation circuit is arranged to control electric load of the piezoelectric transducer between a first load state and a second load state in response to the one-bit data stream, wherein the at least one electric switch is controlled so as to provide different electric loads of the piezoelectric transducer in the first load state than in the second load state.

7. The micro device according to claim 6, wherein in the first load state the at least one electric switch is controlled to short-circuit the terminals of the piezoelectric transducer.

8. The micro device according to claim 6 or 7, wherein in the second load state the at least one electric switch is controlled to provide an electric load of the terminals of the piezoelectric transducer to cause a minimal backscattering from the piezoelectric transducer.

9. The micro device according to any of the preceding claims, wherein the time-encoding analog-to-digital converter is a delta-sigma modulator.

10. The micro device according to any of the preceding claims, wherein the sensor is one of: a neural activity sensor such as a Local Field Potential sensor or a single cell sensor, a bio-chemical sensor, a temperature sensor, or a pressure sensor.

11. The micro device according to any of the preceding claims, being configured for implantation into brain tissue and/or wherein the micro device has a total volume of less than 1 mm$^3$, such as less than 0.5 mm$^3$, such as less than 0.2 mm$^3$.

12. A sensor system comprising

- a micro device (MD1, MD2) according to any of claims 1-11,
- an ultrasonic transmitter (IF2) arranged to transmit an ultrasonic power (UPW) signal to the micro device (MD1, MD2), and
- an ultrasonic receiver (IF2) arranged to receive the backscattered signal (B_U1, B_U2) from the piezoelectric transducer of the micro device (MD1, MD2), and to de-modulate the backscattered signal (U_B1, U_B2), to arrive at a representation of a time sequence of the physical parameter (S1, S2) measured by the sensor in the micro device (MD1, MD2).

13. The sensor system according to claim 12, comprising a plurality of micro devices (MD1, MD2) according to any of claims 1-11, wherein the sensor in each of the plurality of micro devices (MD1, MD2) comprises a neural activity sensor, and wherein the ultrasonic receiver (IF2) is arranged to receive backscattered signals (U_B1, U_B2) from the plurality of micro devices (MD1, MD2), and to de-modulate the backscattered signals to arrive at representations of respective time sequences of neural activities (S1, S2) measured by the plurality of micro devices (MD1, MD2).

14. A method for transmitting sensor data from a micro device implanted in biological tissue, the method comprises

- providing (P_MD) a micro device comprising a piezoelectric transducer connected to a power management circuit for powering power consuming components of the micro device by means of an ultrasonic power signal received by the piezoelectric transducer from an external source, the micro device further comprising a sensor arranged to measure a physical parameter and to generate an electric signal accordingly,
- digitizing (D_ESS) the electric signal from the sensor to generate a one-bit data stream being a representation of the electric signal from the sensor, and
- modulating (M_EL_PZ) electric load of the piezoelectric transducer in response to the one-bit data stream, so that a backscattered signal from the piezoelectric transducer is modulated by the one-bit data stream,

**characterized by**

- storing a time sequence of the generated one-bit data stream in a memory, and applying the stored time sequence of the one-bit data stream to a load modulation circuit at an increased data rate to provide a time compression of data represented in the backscattered signal.

**Patentansprüche**

1. Mikrovorrichtung (MD), die zur Implantation in biologisches Gewebe angeordnet ist, wobei die Mikrovorrichtung (MD) Folgendes umfasst

einen piezoelektrischen Transducer (PZC),
eine Leistungsverwaltungsschaltung (PMC), die mit dem piezoelektrischen Transducer verbunden ist und zum Erzeugen einer elektrischen Ausgangsleistung (PW) zum Versorgen von Komponenten der Mikrovorrichtung (MD) als Reaktion auf ein Ultraschall-Leistungssignal (UPW) angeodrnet ist, das durch den piezoelektrischen Transducer (PZC) von einer externen Quelle empfangen wird,
einen Sensor (SNS), der zum Messen eines physikalischen Parameters oder einer neuronalen Aktivität (LFP) und zum Erzeugen eines entsprechenden elektrischen Signals angeordnet ist,
eine elektrische Schaltung (FE), die zum Empfangen des elektrischen Signals von dem Sensor und zum Digitalisieren des elektrischen Signals durch einen zeitcodierenden Analog-Digital-Wandler (D1B) angeordnet ist, um einen das elektrische Signal von dem Sensor repräsentierenden Ein-Bit-Datenstrom zu erzeugen, und
eine Lastmodulationsschaltung (LMC), die mindestens einen elektrischen Schalter (M3, M4) umfasst, der mit Anschlüssen des piezoelektrischen Transducers (PZC) verbunden ist, um eine Modulation einer elektrischen Last des piezoelektrischen Transducers (PZC) als Reaktion auf den Ein-Bit-Datenstrom zu ermöglichen, sodass ein rückgestreutes Signal (B_U) von dem piezoelektrischen Transducer (PZC) durch den Ein-Bit-Datenstrom moduliert wird, **dadurch gekennzeichnet, dass**
die Mikrovorrichtung (MD) zum Speichern einer Zeitsequenz des erzeugten Ein-Bit-Datenstroms in einem Speicher angeordnet ist, und wobei die gespeicherte Zeitsequenz des Ein-Bit-Datenstroms auf die Lastmodulationsschaltung mit einer erhöhten Datenrate angewendet wird, um eine Zeitkompression von in dem rückgestreuten Signal repräsentierten Daten bereitzustellen.

2. Mikrovorrichtung nach Anspruch 1, wobei die Lastmodulationsschaltung zum Steuern des mindestens einen elektrischen Schalters entsprechend dem Ein-Bit-Datenstrom mit einer Modulationsfrequenz angeordnet ist.

3. Mikrovorrichtung nach Anspruch 1, wobei die erhöhte Datenrate verglichen mit einer Datenrate des Ein-Bit-Datenstroms, der von dem zeitcodierenden Analog-Digital-Wandler erzeugt wird, mindestens einen Faktor 5 aufweist.

4. Mikrovorrichtung nach Anspruch 1, wobei die Mikrovorrichtung zum kontinuierlichen Speichern von Messdaten von dem Sensor über einen bestimmten Zeitraum und zum Abrufen der gespeicherten Daten und Anwenden der Daten auf die Lastmodulationsschaltung während eines Kommunikationszeitraums angeordnet ist.

5. Mikrovorrichtung nach einem der vorhergehenden Ansprüche, wobei die Lastmodulationsschaltung mindestens zwei elektrische Schalter umfasst, die mit dem piezoelektrischen Transducer verbunden und zum Gesteuertwerden angeordnet sind, um die elektrische Last des piezoelektrischen Transducers als Reaktion auf den Ein-Bit-Datenstrom zu modulieren.

6. Mikrovorrichtung nach einem der vorhergehenden Ansprüche, wobei die Lastmodulationsschaltung zum Steuern der elektrischen Last des piezoelektrischen Transducers zwischen einem ersten Lastzustand und einem zweiten Lastzustand als Reaktion auf den Ein-Bit-Datenstrom angeordnet ist, wobei der mindestens eine elektrische Schalter in dem ersten Lastzustand zum Bereitstellen von anderen elektrischen Lasten des piezoelektrischen Transducers als in dem zweiten Lastzustand gesteuert ist.

7. Mikrovorrichtung nach Anspruch 6, wobei der mindestens eine elektrische Schalter in dem ersten Lastzustand zum Kurzschließen der Anschlüsse des piezoelektrischen Transducers gesteuert ist.

8. Mikrovorrichtung nach Anspruch 6 oder 7, wobei der mindestens eine elektrische Schalter in dem zweiten Lastzustand zum Bereitstellen einer elektrischen Last an den Anschlüssen des piezoelektrischen Transducers gesteuert ist, um ein minimales Rückstreuen von dem piezoelektrischen Transducer zu bewirken.

9. Mikrovorrichtung nach einem der vorhergehenden Ansprüche, wobei der zeitcodierende Analog-Digital-Wandler ein Delta-Sigma-Modulator ist.

10. Mikrovorrichtung nach einem der vorhergehenden Ansprüche, wobei der Sensor einer der Folgenden ist: ein Sensor für neuronale Aktivität, wie ein Sensor für ein lokales Feldpotential oder ein Einzelzellsensor, ein biochemischer Sensor, ein Temperatursensor oder ein Drucksensor.

11. Mikrovorrichtung nach einem der vorhergehenden Ansprüche, die für die Implantation in Hirngewebe konfiguriert ist und/oder wobei die Mikrovorrichtung ein Gesamtvolumen von weniger als 1 mm³, wie weniger als 0,5 mm³, wie weniger als 0,2 mm³ aufweist.

12. Sensorsystem, umfassend:

einer Mikrovorrichtung (MD1, MD2) nach einem der Ansprüche 1-11, einen Ultraschallsender (IF2), der zum Senden eines Ultraschall-Leistungssignals (UPW) an die Mikrovorrichtung (MD1, MD2) angeordnet ist, und einen Ultraschallempfänger (IF2), der zum Empfangen des rückgestreuten Signals (B_U1, B_U2) von dem piezoelektrischen Transducer der Mikrovorrichtung (MD1, MD2) und zum Demodulieren des rückgestreuten Signals (U_B1, U_B2) angeordnet ist, um zu einer Repräsentation einer Zeitsequenz des von dem Sensor in der Mikrovorrichtung (MD1, MD2) gemessenen physikalischen Parameters (S1, S2) zu gelangen.

13. Sensorsystem nach Anspruch 12, umfassend eine Vielzahl von Mikrovorrichtungen (MD1, MD2) nach einem der Ansprüche 1-11, wobei der Sensor in jeder der Vielzahl von Mikrovorrichtungen (MD1, MD2) einen Sensor für neuronale Aktivität umfasst und wobei der Ultraschallempfänger (IF2) zum Empfangen rückgestreuter Signale (U_B1, U_B2) von der Vielzahl von Mikrovorrichtungen (MD1, MD2) und zum Demodulieren der rückgestreuten Signale angeordnet ist, um zu Repräsentationen jeweiliger Zeitsequenzen von der Vielzahl von Mikrovorrichtungen (MD1, MD2) gemessener neuraler Aktivitäten (S1, S2) zu gelangen.

14. Verfahren zum Senden von Sensordaten von einer in biologischem Gewebe implantierten Mikrovorrichtung, wobei das Verfahren Folgendes umfasst

Bereitstellen (P_MD) einer Mikrovorrichtung, umfassend einen piezoelektrischen Transducer, der zum Versorgen von Leistung verbrauchenden Komponenten der Mikrovorrichtung durch ein Ultraschallenergiesignal, das durch den piezoelektrischen Transducer von einer externen Quelle empfangen wird, mit einer Leistungsverwaltungsschaltung verbunden ist, wobei die Mikrovorrichtung ferner einen Sensor umfasst, der zum Messen eines physikalischen Parameters und entsprechenden Erzeugen eines elektrisches Signals angeordnet ist, Digitalisieren (D_ESS) des elektrischen Signals von dem Sensor zum Erzeugen eines Ein-Bit-Datenstroms, der eine Repräsentation des elektrischen Signals von dem Sensor ist, und Modulieren (M_EL_PZ) einer elektrischen Last des piezoelektrischen Transducers als Reaktion auf den Ein-Bit-Datenstrom, sodass ein rückgestreutes Signal von dem piezoelektrischen Transducer durch den Ein-Bit-Datenstrom moduliert wird, **gekennzeichnet durch** Speichern einer Zeitsequenz des erzeugten Ein-Bit-Datenstroms in einem Speicher und Anwenden der gespeicherten Zeitsequenz des Ein-Bit-Datenstroms auf eine Lastmodulationsschaltung mit einer erhöhten Datenrate, um eine Zeitkompression von in dem rückgestreuten Signal repräsentierten Daten bereitzustellen.

## Revendications

1. Micro-dispositif (MD), agencé pour être implanté dans un tissu biologique, le micro-dispositif (MD) comprenant

- un transducteur piézoélectrique (PZC),
- un circuit de gestion de puissance (PMC) connecté au transducteur piézoélectrique, et agencé pour générer une sortie de puissance électrique (PW) pour alimenter des composants du micro-dispositif (MD) en réponse à un signal de puissance ultrasonore (UPW) reçu par le transducteur piézoélectrique (PZC) provenant d'une source externe,
- un capteur (SNS) agencé pour mesurer un paramètre physique ou une activité neuronale (LFP), et pour générer un signal électrique en conséquence,
- un circuit électrique (FE) agencé pour recevoir le signal électrique provenant du capteur et pour numériser le signal électrique au moyen d'un convertisseur analogique-numérique à codage temporel (D1B), pour générer un flux de données d'un bit représentant le signal électrique provenant du capteur, et
- un circuit de modulation de charge (LMC) comprenant au moins un commutateur électrique (M3, M4) connecté aux bornes du transducteur piézoélectrique (PZC), de manière à permettre la modulation d'une charge électrique du transducteur piézoélectrique (PZC) en réponse au flux de données d'un bit, de sorte qu'un signal rétrodiffusé (B_U) provenant du transducteur piézoélectrique (PZC) soit modulé par le flux de données d'un bit,

**caractérisée en ce que**
le micro-dispositif (MD) est agencé pour stocker une séquence temporelle du flux de données d'un bit généré dans une mémoire, et appliquer la séquence temporelle stockée du flux de données d'un bit au circuit de modulation de charge à un débit de données accru pour fournir une compression temporelle de données représentées dans le signal rétrodiffusé.

2. Micro-dispositif selon la revendication 1, dans lequel le circuit de modulation de charge est agencé pour commander l'au moins un commutateur électrique en fonction du flux de données d'un bit à une fréquence de modulation.

3. Micro-dispositif selon la revendication 1, dans lequel le débit de données accru est au moins un facteur 5, par rapport à un débit de données du flux de données d'un bit généré par le convertisseur analogique-numérique à codage temporel.

4. Micro-dispositif selon la revendication 1, dans lequel le micro-dispositif est agencé pour stocker en continu des données mesurées provenant du capteur pendant une certaine période, et pour rappeler les données stockées et appliquer les données au circuit de modulation de charge pendant une période de communication.

5. Micro-dispositif selon l'une quelconque des revendications précédentes, dans lequel le circuit de modulation de charge comprend au moins deux commutateurs électriques connectés au transducteur piézoélectrique et agencés pour être commandés afin de moduler une charge électrique du transducteur piézoélectrique en réponse au flux de données d'un bit.

6. Micro-dispositif selon l'une quelconque des revendications précédentes, dans lequel le circuit de modulation de charge est agencé pour commander une charge électrique du transducteur piézoélectrique entre un premier état de charge et un second état de charge en réponse au flux de données d'un bit, dans lequel l'au moins un commutateur électrique est commandé de manière à fournir des charges électriques différentes du transducteur piézoélectrique dans le premier état de charge et dans le second état de charge.

7. Micro-dispositif selon la revendication 6, dans lequel dans le premier état de charge, l'au moins un commutateur électrique est commandé pour court-circuiter les bornes du transducteur piézoélectrique.

8. Micro-dispositif selon la revendication 6 ou 7, dans lequel, dans le second état de charge, l'au moins un commutateur électrique est commandé pour fournir une charge électrique aux bornes du transducteur piézoélectrique afin de provoquer une rétrodiffusion minimale à partir du transducteur piézoélectrique.

9. Micro-dispositif selon l'une quelconque des revendications précédentes, dans lequel le convertisseur analogique-numérique à codage temporel est un modulateur delta-sigma.

10. Micro-dispositif selon l'une quelconque des revendications précédentes, dans lequel le capteur est l'un : d'un capteur d'activité neuronale tel qu'un capteur de potentiel de champ local ou un capteur à cellule unique, d'un capteur biochimique, d'un capteur de température ou d'un capteur de pression.

11. Micro-dispositif selon l'une quelconque des revendications précédentes, étant configuré pour une implantation dans un tissu cérébral et/ou dans lequel le micro-dispositif a un volume total inférieur à 1 mm$^3$, par exemple inférieur à 0,5 mm$^3$, par exemple inférieur à 0,2 mm$^3$.

12. Système de capteur comprenant

    - un micro-dispositif (MD1, MD2) selon l'une quelconque des revendications 1 à 11,
    - un émetteur d'ultrasons (IF2) agencé pour transmettre un signal de puissance ultrasonore (UPW) au micro-dispositif (MD1, MD2), et
    - un récepteur d'ultrasons (IF2) agencé pour recevoir le signal rétrodiffusé (B_U1, B_U2) provenant du transducteur piézoélectrique du micro-dispositif (MD1, MD2), et pour démoduler le signal rétrodiffusé (U_B1, U_B2), pour arriver à une représentation d'une séquence temporelle du paramètre physique (S1, S2) mesuré par le capteur dans le micro-dispositif (MD1, MD2).

13. Système de capteur selon la revendication 12, comprenant une pluralité de micro-dispositifs (MD1, MD2) selon l'une quelconque des revendications 1 à 11, dans lequel le capteur dans chacun de la pluralité de micro-dispositifs (MD1, MD2) comprend un capteur d'activité neuronale, et dans lequel le récepteur d'ultrasons (IF2) est agencé pour recevoir des signaux rétrodiffusés (U_B1, U_B2) provenant de la pluralité de micro-dispositifs (MD1, MD2), et pour démoduler les signaux rétrodiffusés pour arriver à des représentations de séquences temporelles respectives d'activités neuronales (S1, S2) mesurées par la pluralité de micro-dispositifs (MD1, MD2).

14. Procédé de transmission de données de capteur à partir d'un micro-dispositif implanté dans un tissu biologique, le procédé comprend

- la fourniture (P_MD) d'un micro-dispositif comprenant un transducteur piézoélectrique connecté à un circuit de gestion de puissance pour alimenter des composants consommateurs de puissance du micro-dispositif au moyen d'un signal de puissance ultrasonore reçu par le transducteur piézoélectrique provenant d'une source externe, le micro-dispositif comprenant également un capteur agencé pour mesurer un paramètre physique et pour générer un signal électrique en conséquence,
- la numérisation (D_ESS) du signal électrique du capteur pour générer un flux de données d'un bit étant une représentation du signal électrique provenant du capteur, et
- la modulation (M_EL_PZ) d'une charge électrique du transducteur piézoélectrique en réponse au flux de données d'un bit, de sorte qu'un signal rétrodiffusé provenant du transducteur piézoélectrique soit modulé par le flux de données d'un bit, **caractérisé par**
- le stockage d'une séquence temporelle du flux de données d'un bit généré dans une mémoire, et l'application de la séquence temporelle stockée du flux de données d'un bit à un circuit de modulation de charge à un débit de données accru pour fournir une compression temporelle de données représentées dans le signal rétrodiffusé.

FIG. 1a

FIG. 1b

FIG. 2

FIG. 3

Time (μs)

FIG. 4

FIG. 5

FIG. 6

EP 4 208 251 B1

FIG. 7

FIG. 8

**EP 4 208 251 B1**

**Patent documents cited in the description**

- WO 2018009905 A
- WO 2020142733 A
- US 2019321640 A
- WO 2020047152 A